# EUROPEAN PATENT APPLICATION

(11) **EP 3 888 704 A1**
(43) Date of publication of application: **06.10.2021**
(21) Application number: 20167999.0
(22) Date of filing: 03.04.2020
(51) Int. Cl.: A61L 2/24, A61L 2/26, B65G 17/00, B65G 39/00, B25J 5/00, B25J 9/00, A61L 2/12, B25J 15/02, B25J 15/08

(54) **SYSTEM FOR OPENING AND/OR CLOSING A LID OF A CONTAINER CONTAINING MEDICAL EQUIPMENT IN A STERILE PROCESSING DEPARTMENT**

(71) Applicant: Gibotech A/S, 5220 Odense SØ (DK)
(72) Inventor: Anker, Henrik, Odense SØ (DK)
(74) Representative: AWA Denmark A/S

(57) **Abstract**

A system for opening and/or closing a lid of a container containing medical equipment in a sterile processing department, the system comprises: a gripper for opening and/or closing the lid of the container, one or more motorized conveyor elements configured to receive said container at a first container position and convey said container along a longitudinal axis towards the gripper. The system is configured to align the container with the gripper by conveying the container from the first container position to a second container position using the one or more motorized conveyor elements, move a first gripper element and a second gripper element from a first gripper position to a second gripper position, whereby the first gripper element or the second gripper element contacts the container at a side surface of the container and moves the container to a third container position.

## Description

### Field

The present disclosure relates to a system for opening and/or closing a lid of a container containing medical equipment in a sterile processing department, and to a sterile processing department comprising a system for opening and/or closing a lid of a container containing medical equipment.

### Background

Increasing healthcare cost is a global problem which every country faces. Automation of manual process in hospitals can be part of the solution. However, due the high complexity of hospitals the degree of automation until now has been low.

Cleaning and sterilizing of medical equipment such as endoscopes and surgical instruments in a sterile processing department is an example of a work intensive process. Today used medical equipment is typically provided to the sterile processing department in containers, where the containers are manually opened to load and unload medical equipment from the containers. This process requires careful handling of the container, which sometimes may contain contaminated medical instruments, thereby also posing a potential risk for personnel handling the containers.

Thus it remains a problem to provide a simpler and safer system that can aid in automating processes in a sterile processing department and further improve a work environment for personnel.

### Summary

According to a first aspect, the disclosure relates to a system for opening and/or closing a lid of a container containing medical equipment in a sterile processing department, the system comprises:
- a gripper for opening and/or closing the lid of the container, the gripper comprising a first gripper element and a second gripper element, the gripper being configured to move the first gripper element and the second gripper element from a first gripper position to a second gripper position, where the first gripper element and the second gripper element are closer to each other in the second gripper position than in the first gripper position,
- one or more motorized conveyor elements configured to receive said container at a first container position and convey said container along a longitudinal axis towards the gripper,
wherein the system is configured to
align the container with the gripper by conveying the container from the first container position to a second container position using the one or more motorized conveyor elements, where the container in the second container position is aligned with the gripper with a first degree of precision,
move the first gripper element and the second gripper element from the first gripper position to the second gripper position, whereby the first gripper element or the second gripper element contacts the container at a side surface of the container and moves the container to a third container position aligned with the gripper with a second degree of precision, the second degree of precision being higher than the first degree of precision,
open or close the lid of the container in the third container position using the gripper.

Consequently, by providing a system with a gripper for opening and/or closing a lid of a container, a degree of automation may be introduced into a sterile processing department in a simple manner. Automation of the opening and/or closing of the lid of the container may lessen the work cost and personnel associated with handling of containers in the sterile processing department. The gripper may also lessen the amount of human interaction needed with the container, thereby lessening the risk of contaminating personnel and medical equipment. Furthermore, by using a combination of motorized conveyor elements and the gripper for aligning the container, a simple system for handling and aligning containers in a sterile process department is provided.

The gripper may comprise a processing unit. The processing unit may be any unit which comprises a unit able to perform basic arithmetic, such as a central processing unit (CPU) or a graphics processing unit (GPU). The gripper may comprise sensors and/or cameras in order to orientate itself and/or to assess distances. The gripper may comprise force sensors allowing the gripper to assess if contact have been made to the container. The gripper may comprise means for lifting the lid of the container, e.g. suction cups and/or gripper elements being configured for lifting the lid of the container.

The first gripper element and the second gripper element may be identical or differ from each other. The first gripper element and the second gripper element may be made out of easy to clean material such as stainless steel or aluminum. At least some edges of the first gripper element and the second gripper element may be chamfered to avoid scratching and/or damaging of the container.

The gripper is configured to move the first gripper element and the second gripper element between a first gripper position and a second gripper position. Where the first gripper element and the second gripper element are closer to each other in the second gripper position than in the first gripper position. In the first gripper position the first gripper element and the second gripper element may have a distance between each other longer than a width or a length of the container. In the second gripper position the first gripper element and the second gripper element may have a distance between each other substantially equal to a width or a length of the container.

The one or more motorized conveyor elements may be a motorized roller(s), belt or chain conveyor. The motorized conveyor elements may be used in conjunction with non-motorized conveyor elements such as non-motorized roller elements.

The container is configured to assume at least three different container positions a first container position, a second container position, and a third container position. The first container position being a position in which the container is received on the one or more motorized conveyor elements, and is ready to be conveyed along a longitudinal axis. The container may be transferred to the first container position via personnel, a robot arm or an autonomous transport vehicle. The second container position being a position where the container is aligned with the gripper with a first degree of precision. The alignment of the container may happen via the one or more motorized conveyor elements conveying the container to the second container position. The third container position being a position wherein the container is aligned with the gripper with a second degree of precision. The container may be aligned with the second degree of precision by the first gripper element or the second gripper element contacting and moving the container from the second container position to the third container position.

The first degree of precision may be a deviation in position the container exhibits, with regards to being aligned with the gripper. The first degree of precision may be a deviation in position along a lateral axis, or a longitudinal axis. The lateral axis being an axis perpendicular to the longitudinal axis, and extending in the horizontal plane. In some cases, where the first degree of precision is a deviation in container position along the lateral axis, with regards to being aligned with the gripper, the container may already be aligned with the first degree of precision when transferred to the first container position. In other cases, where the first degree of precision is a deviation in container position along the longitudinal axis, with regards to being aligned with the gripper, the container may firstly be aligned with the first degree of precision when conveyed to the second container position by the one or more motorized conveyor elements.

The second degree of precision may be a deviation in position the container exhibits, with regard to being aligned with the gripper. The second degree of precision may be a deviation in position along a lateral axis, or a longitudinal axis. The second degree precision having a higher degree of precision than the first degree of precision, e.g. a smaller deviation in position along either the longitudinal or lateral axis. In some cases, where the second degree of precision is a deviation in container position along the lateral axis, with regards to being aligned with the gripper, the first gripper element or the second gripper element may contact the container and move it along the lateral axis to align the container with the second degree of position. In other cases, where the second degree of precision is a deviation in container position along the longitudinal axis, with regards to being aligned with the gripper, the first gripper element or the second gripper element may contact the container and move it along the longitudinal axis to align the container with the second degree of position.

The container may be a sterilizing container, e.g. a container configured to support the medical equipment in an autoclave. The sterilizing container may be provided with a lid allowing a sterilizing fluid e.g. hot steam, to enter the container. The lid may further be configured to form a fluid tight seal when the sterilizing container cools down whereby the medical equipment within the medical container may be stored under sterile conditions.

In some embodiments the system comprises a pressure element configured to apply a pressure to a top surface of the container.

Having a pressure element may keep the container in place after it has been aligned with the gripper with the second degree of precision. Thereby, assuring the container does not move unnecessarily while being opened or closed. The pressure element may be any element capable of delivering a pressure, such as a weight in connection with a piston.

In some embodiments the system comprises one or more flippers configured to assist with aligning the container with the gripper.

The one or more flippers may be motorized flippers or resiliently loaded flippers. The one or more flippers being configured to push a container either along the longitudinal axis or along the lateral axis, or to rotate the container to align an orientation of the container.

In some embodiments the system the first gripper element and the second gripper element are synchronized to move simultaneously and at the same rate between the first gripper position and the second gripper position.

In some embodiments the system comprises a motor, wherein the first gripper element and the second gripper element are configured to be moved between the first gripper position and the second gripper position by the motor.

Consequently, a simple system wherein the first gripper element and the second gripper element need only one motor to move. Thereby, points of failure within the system is minimized. The motor may be an electric motor such as a stepper motor. The motor may alternatively be a hydraulic motor.

In some embodiments the gripper is configured to move up and down along a vertical axis perpendicular to the longitudinal axis.

Consequently, this may allow the gripper to adjust for different containers, and to adjust in-between containers.

In some embodiments the gripper is configured to assume a first vertical position along the vertical axis when moving the first gripper element and the second gripper element from the first gripper position to the second gripper position, and to assume a second vertical position along the vertical axis when opening or closing the lid of the container.

In some embodiments the first gripper element and the second gripper element are placed along an orientation axis, where the orientation axis is either parallel to the longitudinal axis or perpendicular to the longitudinal axis.

Dependent on the orientation of the container when received at the first container position, it may be advantageous to have the first gripper element and the second gripper element with a matching orientation in order to open/close the container, e.g. an orientation matching that of an opening mechanism of the container.

In some embodiments, where the orientation axis is parallel to the longitudinal axis, the gripper is configured to assume a third vertical position along the vertical axis and to assume a fourth vertical position along the vertical axis, where in the third vertical position the gripper is configured to engage a side surface of the container, and where in the fourth vertical position the gripper is raised above the container.

Being able to raise the gripper above the container may help preventing containers from clogging up the motorized conveyor elements, by assuring the containers are free to pass the gripper underneath the gripper. Alternatively, the gripper may be configured to move transversely, to a first transverse position where the gripper is configured to engage a side surface of the container, and to a second transverse position where the gripper is moved to a side of the container.

In some embodiments the system is further configured to open and/or close a lid of a first container type and a second container type, the system being configured to determine whether the container received at the first container position is the first container type or the second container type, and
wherein the opening or closing of the lid of the container is done in correspondence to the type of the container determined by the system.

Consequently, the gripper may adapt to a container before trying to open or close it by determining the type of container. This is especially advantageous if the system is to be in a sterile processing department with different types of containers. Although, only a first and a second type is mentioned, the system may also be configured to determine a third type, a fourth type, a fifth type, and etc.

The system may be able to determine the type of container through a plethora option. In one case the type of container is determined by an operator inputting the type of container to the system. The system may receive a data log where from the type of container is evident. The containers may be provided with tags, such as QR-codes, bar codes, RFID, or a combination of these, the tags comprising information about the container, such as the container type. The system may be provided with a reader for reading the tags, thereby determining the type of container. The system may alternatively or in combination be provided with cameras for determining the type of container.

In some embodiments each of the first gripper element and the second gripper element comprises a hook configured to contact a surface of the container and subsequently opening or closing the lid of the container.

In some embodiments at least one of the first gripper element and the second gripper element comprises a protrusion configured to contact a surface of the container and subsequently opening or closing the lid of the container.

In some embodiments the system is configured to open or close the container using the protrusions if the first container type is determined or the hooks if the second container type is determined.

In some embodiments the system comprises one or more containers.

According to a second aspect the disclosure relates to use of a system disclosed in relation to the first aspect of the disclosure in a sterile processing department.

According to a third aspect the disclosure relates to a sterile processing department comprising a system disclosed in relation to the first aspect of the disclosure.

The different aspects of the present invention can be implemented in different ways including a transport system, use of a transport system and a sterile processing department described above and in the following, each yielding one or more of the benefits and advantages described in connection with at least one of the aspects described above, and each having one or more preferred embodiments corresponding to the preferred embodiments described in connection with at least one of the aspects described above and/or disclosed in the dependent claims. Furthermore, it will be appreciated that embodiments described in connection with one of the aspects described herein may equally be applied to the other aspects.

### Brief description of the drawings

The above and/or additional objects, features and advantages of the present invention, will be further elucidated by the following illustrative and non-limiting detailed description of embodiments of the present invention, with reference to the appended drawings, wherein:
Fig. 1 shows a perspective view of an embodiment of the system, wherein a gripper is mounted on a frame
Fig. 2 shows a perspective view of an embodiment of the one or more motorized conveyor elements.
Fig. 3 shows a perspective view of another embodiment of the one or more motorized conveyor elements.
Fig. 4 shows a perspective view of yet another embodiment of the one or more motorized conveyor elements.
Fig. 5 shows a perspective view of an embodiment of the gripper, wherein the first gripper element and the second gripper element are in the first gripper position.
Fig. 6 shows a perspective view of another embodiment of the gripper, wherein the first gripper element and the second gripper element are in the first gripper position.
Fig. 7 shows a perspective view of an embodiment of the system, wherein the orientation axis is parallel to the lateral axis and the first gripper element and the second gripper element are placed along the orientation axis.
Fig. 8 shows a perspective view of an embodiment of the system, wherein the orientation axis is parallel to the longitudinal axis and the first gripper element and the second gripper element are placed along the orientation axis.
Figs. 9a-9d shows a schematic side view of a work flow process of an embodiment of the system.
Figs. 10a-10d shows a schematic side view of a work flow process of another embodiment of the system.

### Detailed description

In the following description, reference is made to the accompanying figures, which show by way of illustration how the invention may be practiced.

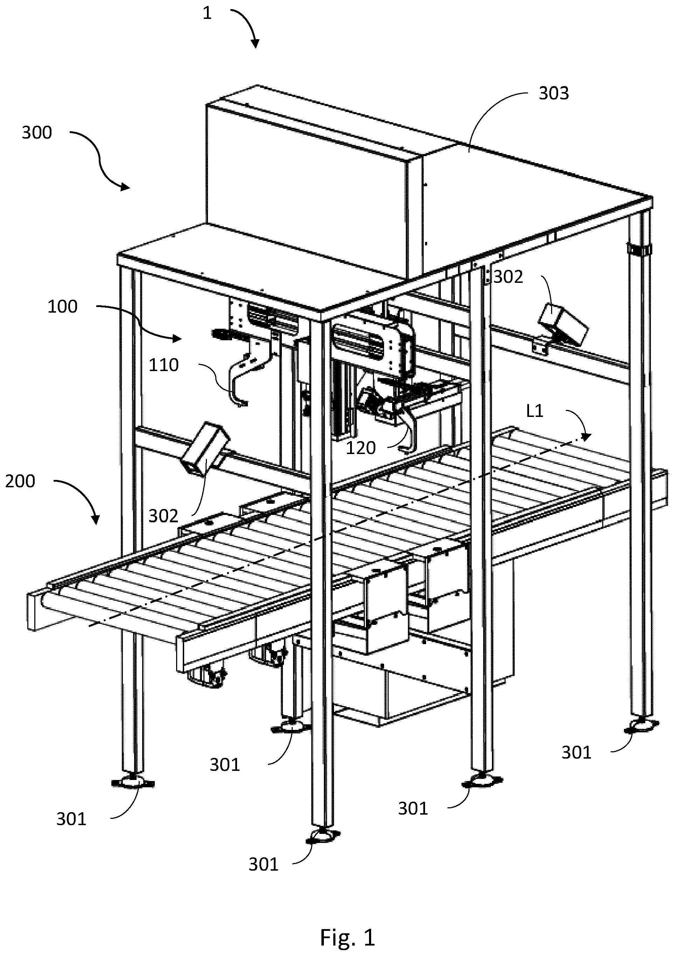

Fig. 1 shows a perspective view of an embodiment of the system 1, wherein a gripper 100 is mounted on a frame 300. The frame 300 may be a modular frame 300 capable of being taken apart and built at a worksite in a sterile processing department. The frame 300 may be built on raiseable or lowerable feet 301, thereby allowing a general height of the frame 300 to be controlled. The frame 300 may be provided with external sensors or readers, e.g. cameras 302, distance sensors, or QR-readers for analysing containers received on the one or more motorized conveyor elements 200. The frame 300 may be provided with a top housing 303, for housing drive means of the gripper 100 or for housing general electronics. The gripper 100 is configured for opening or closing a container received on the one or more motorized conveyor elements 200. The one or more motorized conveyor elements 200 may receive the container at a first container position. Subsequently, the container is conveyed along a longitudinal axis L1 to a second container position, wherein the container is aligned with the gripper 100 with a first degree of precision. The gripper 100 then moves a first gripper element 110 and a second gripper element 120 from a first gripper position to a second gripper position resulting in either the first gripper element 110 or the second gripper element 120 contacting and moving the container to a third container position. The container in the third container position is aligned with the gripper 100 with a second degree of precision. When the container is aligned with the second degree of precision, the gripper 100 may open or close the container.

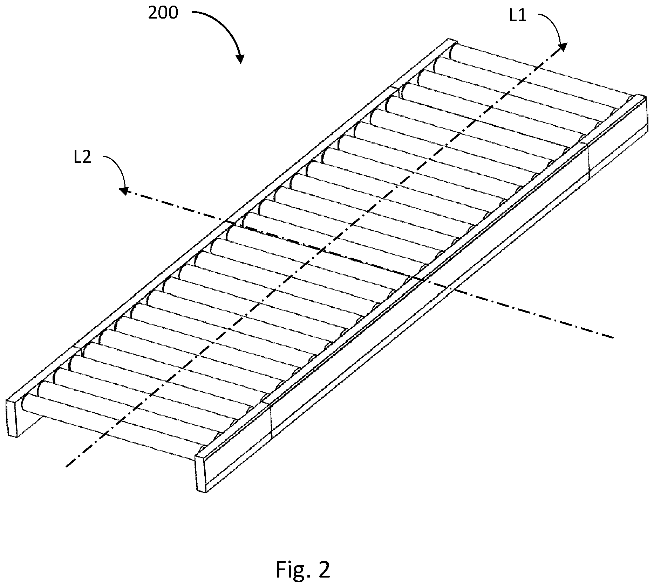

Fig. 2 shows a perspective view of an embodiment of the one or more motorized conveyor elements 200. In the shown embodiment the one or more motorized conveyor elements 200 are formed as a plurality of rollers 200. The rollers 200 extend longitudinally along a lateral axis L2. At least one roller within the plurality of rollers 200 is a motorized roller capable of rotating along its own longitudinal axis. In some embodiments more than one of the rollers 200 are motorized, e.g. two, three, four or all of them. The one or more motorized roller within the plurality of rollers 200 allows for a container received on the rollers 200 to be conveyed along the longitudinal axis L1. Although, rollers are shown in the current embodiment, the one or more motorized conveyor element is not limited to rollers, but may also be a belt, or a chain conveyor.

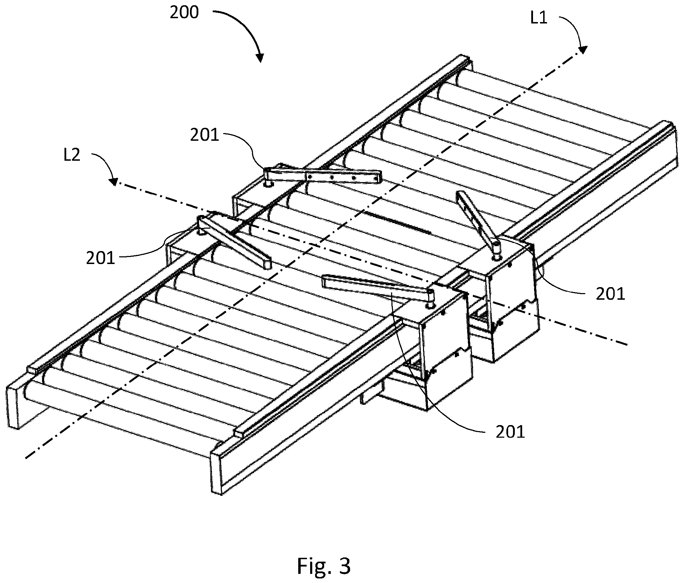

Fig. 3 shows a perspective view of another embodiment of the one or more motorized conveyor elements 200. In the shown embodiment the system further comprises four flippers 201. In other embodiments one flipper, two flippers, or three flippers may be used. The flippers 201 may assist the system 1 in aligning a container received on the one or more motorized conveyor elements 200 with the gripper 100. The flippers 201 may assist the system 1 by pushing the received container, so as to correct an orientation of the container, to push the container along the longitudinal axis L1, and/or to push the container along the lateral axis L2. The flippers 201, may be motorized or resiliently loaded.

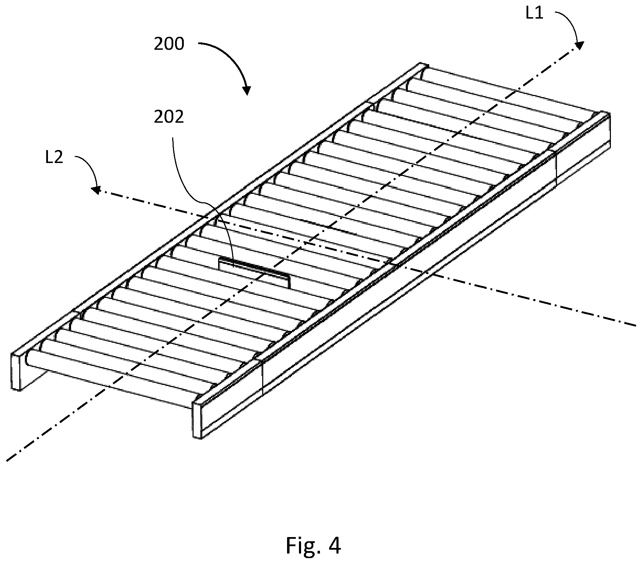

Fig. 4 shows a perspective view of yet another embodiment of the one or more motorized conveyor elements 200. In the shown embodiment the system further comprises a stop plate 202. The stop plate 202 may assist the one or more motorized conveyor elements 200 in aligning a container received on the one or more motorized conveyor elements 200 with the gripper 100. The stop plate 202 may act to stop a container being conveyed along the longitudinal axis L1. By being able to stop a container at a certain position may assist in aligning a container. The stop plate 202 may be configured to be raised and lowered. The stop plate 202 may be configured to stop a container when raised and to allow passage of a container when lowered. Alternatively, or in combination, the system 1 may comprise guide rails for assisting in aligning the container with the gripper. Guide rails may help aligning a received container along the lateral axis L2, e.g. by being formed as a funnel capable of guiding a container being conveyed.

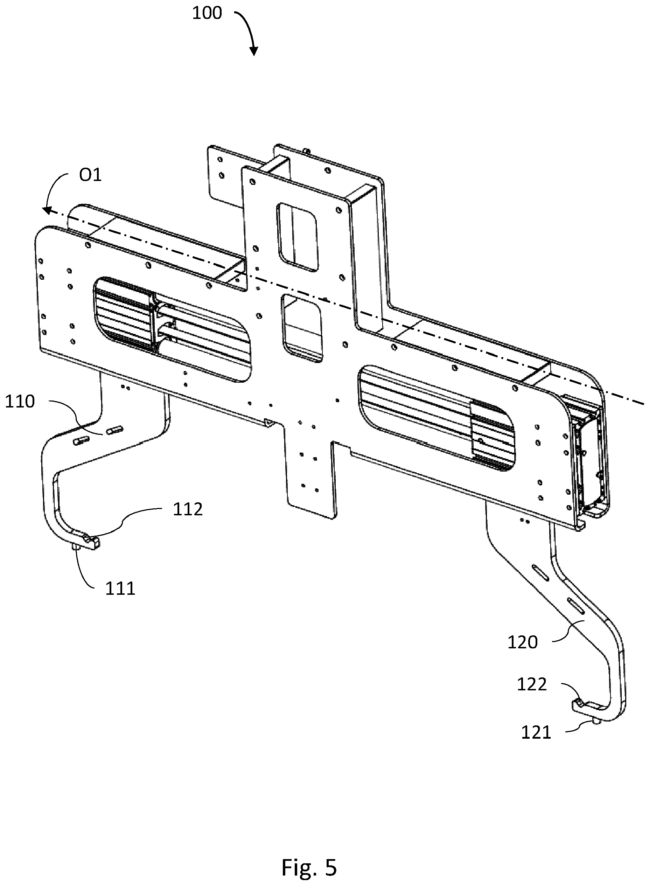

Fig. 5 shows a perspective view of an embodiment of the gripper 100, wherein the first gripper element 110 and the second gripper element 120 are in the first gripper position. The gripper 100 comprises the first gripper element 110 and the second gripper element 120. The first gripper element 110 and the second gripper element 120 may be placed along an orientation axis O1. The first gripper element 110 comprises a first protrusion 111 and a first hook 112. In some embodiments the first gripper element 110 only comprises the first protrusion 111, or the first hook 112. The second gripper element 120 comprises a second protrusion 121 and a second hook 122. In some embodiments the second gripper element 120 only comprises the second protrusion 111, or the second hook 112. The protrusions 111, 121 and the hooks 112, 122 are configured for interacting with an opening mechanism of a container received on the one or more motorized conveyor elements 200. The first gripper element 110 and the second gripper element 120 may be provided with hooks 112, 122 and/or protrusions 111, 121 dependent on types of containers received on the one or more motorized conveyor elements 200. The first gripper element 110 and the second gripper element 120 may also be provided with other opening or closing means than hooks or protrusions, dependent on the opening mechanism of containers received on the one or more motorized conveyor elements 200. The hooks 112, 122 may be configured to interact with hinges on the side of a received container, thereby opening or closing the container via interaction between the hooks 112, 122 and the hinges of the received container. Some containers may be provided with a push-to-open opening mechanism, wherein the protrusions 111, 121 may push down on the push-to-open opening mechanism to open and/or close the container. For containers with the push-to-open opening mechanism it may be enough to only have one of the gripper elements 110, 120 comprising a protrusion. The first gripper element 110 is able to move along the orientation axis O1, thereby allowing the first gripper element 110 to move from the first gripper position to the second gripper position. The first gripper element 110 may move linearly, or pivotally from the first gripper position to the second gripper position. The second gripper element 120 is able to move along the orientation axis O1, thereby allowing the second gripper element 120 to move from the first gripper position to the second gripper position. The second gripper element 120 may move linearly, or pivotally from the first gripper position to the second gripper position. The movement of the first gripper element 110 and the second gripper element 120 from the first gripper position to the second gripper position moves the first gripper element 110 and the second gripper element 120 towards each other.

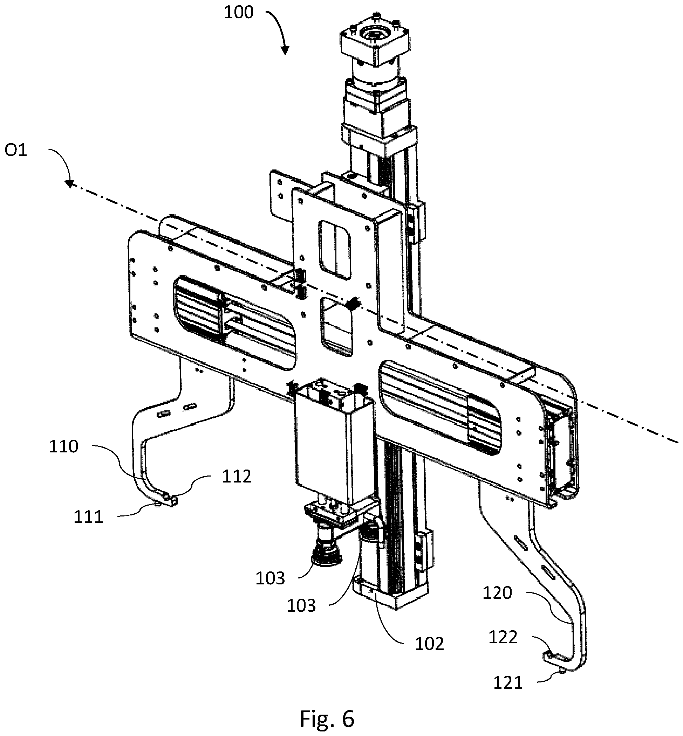

Fig. 6 shows a perspective view of another embodiment of the gripper 100, wherein the first gripper element 110 and the second gripper element 120 are in the first gripper position. The gripper 100 is provided with a pressure element 102 for delivering a pressure to a top surface of a container. The pressure element 102 in the current embodiment is formed as piston with a stamp 102. Alternatively, the pressure element 102 may be any kind of weight which can be raised and lowered. Furthermore, the gripper 100 is provided with suction cups 103. The suction cups 103 may adhere onto a top surface of a container, and lift the lid of the container.

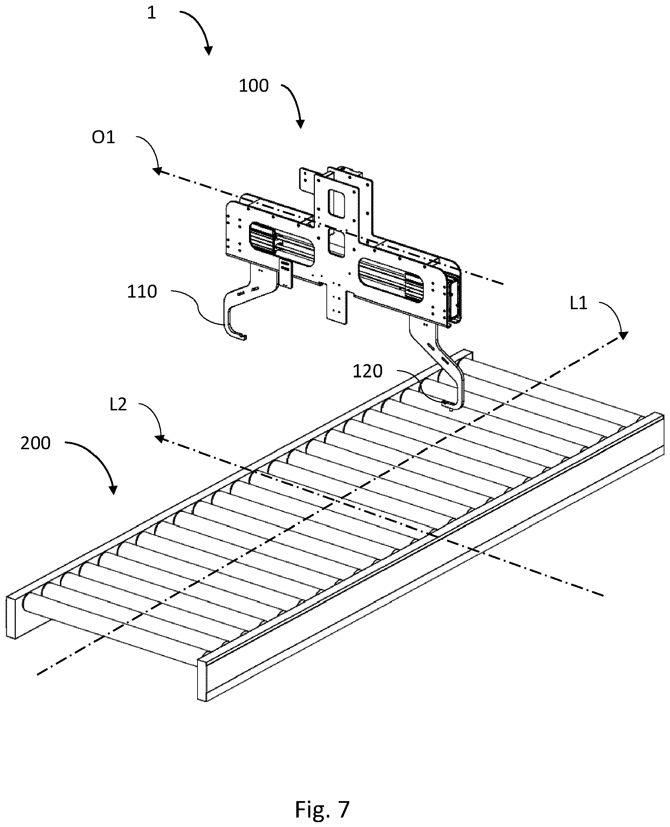

Fig. 7 shows a perspective view of an embodiment of the system 1, wherein the orientation axis O1 is parallel to the lateral axis L2 and the first gripper element 110 and the second gripper element 120 are placed along the orientation axis O1. The gripper may be able to move along a vertical axis V1, e.g. when a container is received the in the second container position the gripper may move down, and subsequently move the first gripper element 110 and the second gripper element 120 from the first gripper position to the second gripper position and open or close the container, after opening/closing the container the gripper 100 may move the first gripper element 110 and the second gripper element 120 back to the first gripper position, and move up to allow the container to pass further along the longitudinal axis L1. The gripper 100 may be configured to assume a first vertical position along the vertical axis when moving the first gripper element 110 and the second gripper element 120 from the first gripper position to the second gripper position, and to assume a second vertical position along the vertical axis when opening or closing the lid of the container. In this case where the first gripper element 110 and the second gripper element are placed along the orientation axis O1 parallel to the lateral axis L2, the first degree of precision may be defined as a deviation in position along the lateral axis, with regards to being aligned with the gripper 100. The container may already have the first degree precision when transferred to the first container position. The second degree of precision may be defined a deviation in position along the lateral axis, with regards to being aligned with the gripper 100, where the second degree of precision is higher than the first degree of precision. When the one or more motorized conveyor elements 200 have conveyed the container to the second container position, the first gripper element 110 or the second gripper element 120 may contact and push the container along the lateral axis L2, thereby aligning the container with the gripper 100 with the second degree of precision. Furthermore, the first gripper element 110 and the second gripper element 120 may also correct an orientation of the container, when moving from the first gripper position to the second gripper position.

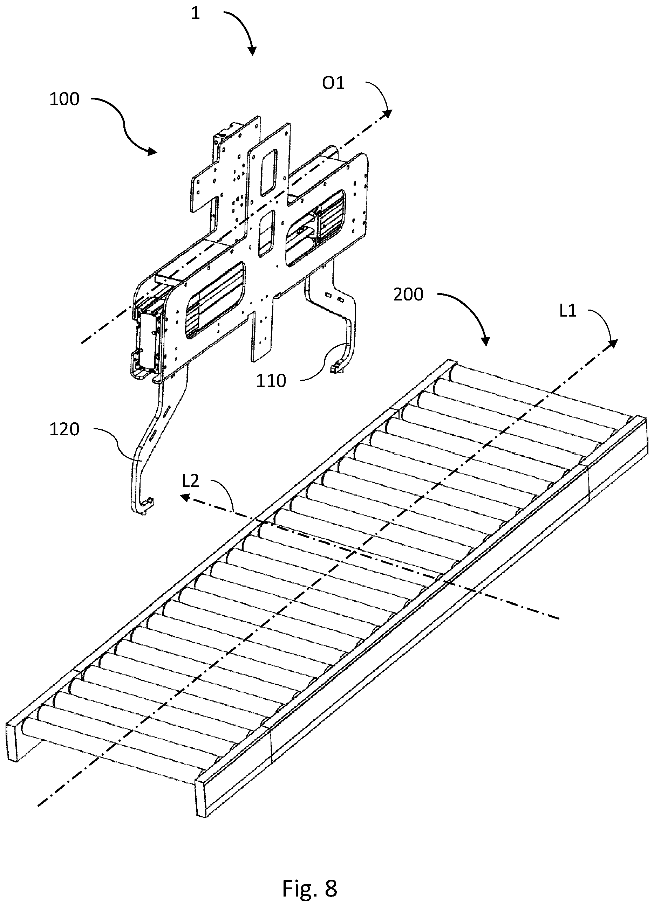

Fig. 8 shows a perspective view of an embodiment of the system 1, wherein the orientation axis O1 is parallel to the longitudinal axis L1 and the first gripper element 110 and the second gripper element 120 are placed along the orientation axis O1. In this case where the first gripper element 110 and the second gripper element are placed along the orientation axis O1 parallel to the longitudinal axis L1, the first degree of precision may be defined as a deviation in position along the longitudinal axis L1, with regards to being aligned with the gripper 100. The container may be aligned with the first degree of position by being conveyed to the second container position by the one or more motorized conveyor element 200. The second degree of precision may be defined as a deviation in position along the longitudinal axis L1, with regards to being aligned with the gripper 100, though with a higher precision than the first degree of precision. When the one or more motorized conveyor elements 200 have conveyed the container to the second container position, the first gripper element 110 or the second gripper element 120 may contact and push the container along the longitudinal axis L1, thereby aligning the container with the gripper 100 with the second degree of precision. Furthermore, the first gripper element 110 and the second gripper element 120 may also correct an orientation of the container, when moving from the first gripper position to the second gripper position.

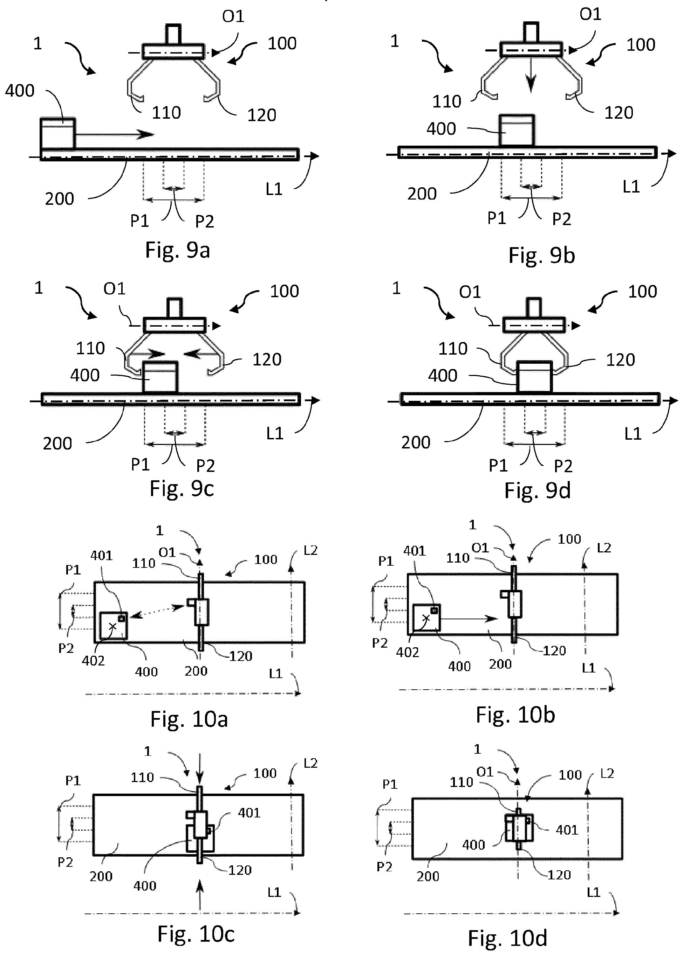

Figs. 9a-9d shows a schematic side view of a work flow process of an embodiment of the system 1. In the shown embodiment the first gripper element 110 and the second gripper element 120 are placed along on an orientation axis O1. The orientation axis O1 being parallel to the longitudinal axis L1. In Fig. 9a a container 400 have been received on the one or motorized conveyor elements 200 at the first container position. The container 400 is then conveyed along the longitudinal axis L1 to the second container position shown on Fig. 9b. At the second container position, the container is aligned with the gripper 100 with the first degree of precision P1, which in the shown embodiment is a deviation in position along the longitudinal axis, with regards to being aligned with the gripper. The lines in the figures illustrating P1 shows where the center point of a container may be arranged after being conveyed by the one or more motorized conveyors e.g. with 4 standard deviations. The gripper 100 is then lowered to allow the first gripper element 110 or the second gripper element 120 to contact a side of the container 400. When the gripper 100 has been lowered, as seen fig. 9c, the gripper 100 moves the first gripper element 110 and the second gripper element 120 from the first gripper position to the second gripper position. The movement of the first gripper element 110 and the second gripper element 120 from the first gripper position to the second gripper position, allows, in the shown example where the one or more motorized conveyor elements 200 have moved the container a bit too short, the first gripper element 110 to contact the container and moves it toward the second gripper element 120. However, for the next container, the one or motorized conveyor elements 200 may move the next container a bit too long, whereby it will be the second gripper element 120 that contacts the container and moves it toward the first gripper element 110. The movement of the container 400 towards the second gripper element 120 results in the container 400 being aligned with the gripper 100 with the second degree of precision P2. The second degree of precision P2 being a deviation in position along the longitudinal axis, with regards to being aligned with the gripper 100. The second degree of precision P2 being higher than the first degree of precision P1. The lines in the figures illustrating P2 shows where the center point of a container may be arranged after being moved by the first gripper element 110 or the second gripper element 120 e.g. with 4 standard deviations.

After the container 400 has been aligned with the second degree of precision, the gripper 100 may either open or close the container 400.

Figs. 10a-10d shows a schematic side view of a work flow process of another embodiment of the system 1. In the shown embodiment the first gripper element 110 and the second gripper element 120 are placed along on an orientation axis O1. The orientation axis O1 being parallel to the lateral axis L2.
In Fig. 10a a container 400 have been received on the one or motorized conveyor elements 200 at the first container position. The container 400 is provided with a container tag 401. The container tag 401 may be a QR-code, an RFID-chip, a barcode, etc. The container tag 401 comprising container information about the container 400. The container information comprising information on what type of container it is. The system comprises a reader 500 for reading the container tag 401. At the first container position the reader 500 reads the container tag 401, thereby determining the type of container. Alternatively, the reader 500 may read the container tag 401 at the second container position or the third container position. After the reader 500 has determined the type of container it is communicated to the gripper 100. In the first container position a center point 402 of the container 400 is aligned with the gripper with a first degree of precision P1. The first degree of precision P1 being a deviation in position along the transversal axis, with regards to being aligned with the gripper 100. The lines in the figures illustrating P1 shows where the center point of a container may be arranged after being received at the first container position e.g. with 4 standard deviations. The container 400 is then conveyed along the longitudinal axis L1 to the second container position shown on Fig. 10c. The gripper 100 then moves the first gripper element 110 and the second gripper element 120 from the first gripper position to the second gripper position. The movement of the first gripper element 110 and the second gripper element 120 from the first gripper position to the second gripper position, allows, in the shown embodiment, the second gripper element 120 to contact the container and move it toward the first gripper element 110. The movement of the container towards the first gripper element 110 results in the center point of the container being aligned with the gripper 100 with the second degree of precision P2. The second degree of precision P2 being a deviation in position along the transversal axis, with regards to being aligned with the gripper 100. The second degree of precision P2 being higher than the first degree of precision P1. After the container 400 has been aligned with the second degree of precision P2, the gripper 100 may either open or close the container 400 in accordance with the type of container communicated to the gripper 100. The lines in the figures illustrating P2 shows where the center point of a container may be arranged after being moved by the first gripper element 110 or the second gripper element 120 e.g. with 4 standard deviations.

Although some embodiments have been described and shown in detail, the invention is not restricted to them, but may also be embodied in other ways within the scope of the subject matter defined in the following claims. In particular, it is to be understood that other embodiments may be utilised and structural and functional modifications may be made without departing from the scope of the present invention.

In device claims enumerating several means, several of these means can be embodied by one and the same item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

## Claims

1. A system for opening and/or closing a lid of a container containing medical equipment in a sterile processing department, the system comprises:
• a gripper for opening and/or closing the lid of the container, the gripper comprising a first gripper element and a second gripper element, the gripper being configured to move the first gripper element and the second gripper element from a first gripper position to a second gripper position, where the first gripper element and the second gripper element are closer to each other in the second gripper position than in the first gripper position,
• one or more motorized conveyor elements configured to receive said container at a first container position and convey said container along a longitudinal axis towards the gripper,
wherein the system is configured to
align the container with the gripper by conveying the container from the first container position to a second container position using the one or more motorized conveyor elements, where the container in the second container position is aligned with the gripper with a first degree of precision,
move the first gripper element and the second gripper element from the first gripper position to the second gripper position, whereby the first gripper element or the second gripper element contacts the container at a side surface of the container and moves the container to a third container position aligned with the gripper with a second degree of precision, the second degree of precision being higher than the first degree of precision,
open or close the lid of the container in the third container position using the gripper.

2. A system according to claim 1, wherein the system further comprises a pressure element configured to apply a pressure to a top surface of the container.

3. A system according to claim 1 or 2, wherein the system further comprises one or more flippers configured to assist aligning the container with the gripper.

4. A system according to any of the preceding claims, wherein the first gripper element and the second gripper element are synchronized to move simultaneously and at the same rate between the first gripper position and the second gripper position.

5. A system according to claim 4, where the system further comprises a motor, wherein the first gripper element and the second gripper element are configured to be moved between the first gripper position and the second gripper position by the motor.

6. A system according to any of the preceding claims, wherein the gripper is configured to move up and down along a vertical axis perpendicular to the longitudinal axis.

7. A system according to claim 6, wherein the gripper is configured to assume a first vertical position along the vertical axis when moving the first gripper element and the second gripper element from the first gripper position to the second gripper position, and to assume a second vertical position along the vertical axis when opening or closing the lid of the container.

8. A system according to any of the preceding claims, wherein the first gripper element and the second gripper element are placed along an orientation axis, where the orientation axis is either parallel to the longitudinal axis or parallel to a lateral axis.

9. A system according to claim 6 and 8, where the orientation axis is parallel to the longitudinal axis, the gripper is configured to assume a third vertical position along the vertical axis and to assume a fourth vertical position along the vertical axis, where in the third vertical position the gripper is configured to engage a side surface of the container, and where in the fourth vertical position the gripper is raised above the container.

10. A system according to any of the preceding claims, wherein the system is further configured to open and/or close a lid of a first container type and a second container type, the system being configured to determine whether the container received at the first container position is the first container type or the second container type, and
wherein the opening or closing of the lid of the container is done in correspondence to the type of the container determined by the system.

11. A system according to any of the preceding claims, wherein each of the first gripper element and the second gripper element comprises a hook configured to contact a surface of the container and subsequently opening or closing the lid of the container.

12. A system according to any of the preceding claims, wherein at least one of the first gripper element and the second gripper element comprises a protrusion configured to contact a surface of the container and subsequently opening or closing the lid of the container.

13. A system according to claims 10-12, wherein the system is configured to open or close the container using the protrusions if the first container type is determined or the hooks if the second container type is determined.

14. Use of a system according to any of claims 1-13 in sterile processing department.

15. A sterile processing department comprising a system according to any one of claims 1-13.
